Europäisches Patentamt

European Patent Office

Office européen des brevets

(19)

(11) EP 0 748 305 B1

(12) **EUROPEAN PATENT SPECIFICATION**

(45) Date of publication and mention
of the grant of the patent:
**16.08.2000 Bulletin 2000/33**

(21) Application number: **95944407.6**

(22) Date of filing: **29.12.1995**

(51) Int Cl.[7]: **C07C 2/70**, C07C 2/64

(86) International application number:
**PCT/US95/16931**

(87) International publication number:
**WO 96/20905 (11.07.1996 Gazette 1996/31)**

(54) **CATALYST AND PROCESS FOR ALKYLATION OF AROMATICS**

KATALYSATOREN UND VERFAHREN ZUR ALKYLIERUNG VON AROMATEN

CATALYSEUR ET PROCEDE POUR ALKYLER DES AROMATIQUES

(84) Designated Contracting States:
**DE FR GB NL**

(30) Priority: **30.12.1994  US 366842**

(43) Date of publication of application:
**18.12.1996  Bulletin 1996/51**

(73) Proprietor: **Chevron Chemical Company LLC
San Francisco, CA 94105 (US)**

(72) Inventors:
• **HARRIS, Thomas, V.
Benicia, CA 94510 (US)**
• **CAMPBELL, Curt, B.
Hercules, CA 94547 (US)**
• **SANTILLI, Donald, S.
Larkspur, CA 94939 (US)**

(74) Representative: **Nash, David Allan
Haseltine Lake & Co.,
Imperial House,
15-19 Kingsway
London WC2B 6UD (GB)**

(56) References cited:
| | |
|---|---|
| WO-A-94/14732 | US-A- 3 932 306 |
| US-A- 4 358 628 | US-A- 4 489 213 |
| US-A- 4 873 017 | US-A- 5 015 786 |
| US-A- 5 300 722 | US-A- 5 396 011 |
| US-A- 5 396 012 | US-A- 5 434 326 |

• **BULLETIN OF THE CHEMICAL SOCIETY OF
JAPAN, 61, pp. 1379-1381, April 1988, R.A.
RAJADHYAKSHA et al., "Alkylation of Phenol by
C9 and C12 Olefins".**

## Description

The present invention relates to a catalyst and process for alkylation of non-oxygen-containing aromatic hydrocarbons, such as benzene, toluene, xylene, or mixtures thereof.

## BACKGROUND OF THE INVENTION

It is well known to catalyze the alkylation of aromatics with a variety of Lewis or Brönsted acid catalysts. Typical commercial catalysts include phosphoric acid/kieselguhr, aluminum chloride, and hydrogen fluoride. Alkylation with lower molecular weight olefins, such as propylene, can be carried out in the liquid or vapor phase. For alkylations with higher olefins, such as $C_{16+}$ olefins, the alkylations are done in the liquid phase, usually in the presence of hydrogen fluoride. Alkylations of benzene with higher olefins is especially difficult, and requires hydrogen fluoride treatment. Such a process is disclosed by Himes in U.S. Patent No. 4,503,277, entitled "HF Regeneration in Aromatic Hydrocarbon Alkylation Process," which is hereby incorporated by reference for all purposes. However, hydrogen fluoride is not environmentally attractive.

The use of these acids is extremely corrosive, thus requiring special handling and equipment. Also, the use of these acids might involve environmental problems. Thus, it would preferable to use a safer, simpler catalyst, preferably in solid state, in a fixed bed reactor. This simpler process would result in less capital investment, which would result in a less expensive product.

A process for using solid catalysts to produce alkyl aromatics using lower olefins ($C_6$ to $C_{22}$) is disclosed by Vora et al. in U.S. Patent No. 5,012,021, entitled "Process for the Production of Alkylaromatic Hydrocarbons Using Solid Catalysts," which is hereby incorporated by reference for all purposes. The disclosed solid catalysts include amorphous silica-alumina, crystalline aluminosilicate materials, naturally occurring and man-made clays, and acidic polymer catalysts. Vora et al. does not mention sulfonated zirconia as a possible catalyst.

The use of sulfated zirconia catalyst for alkylation of phenol is disclosed by Rajadhyaksha et al., in "Alkylation of Phenol by $C_9$ and $C_{12}$ Olefins," Bull. Chem. Soc. Jpn., **61**, 1379-1381 (1988).

WO 94/14732 discloses a process in which an aromatic compound is alkylated with an alkene in the presence of a tungsten or zirconia catalyst.

None of these references recognize that, for the very difficult reaction of alkylation of benzene or alkyl benzene with a high molecular weight olefin ($C_{16}$ to $C_{28}$), metal oxides, such as zirconium oxide or tin oxide, are good catalysts when activated prior to use and when the activated catalyst is used without exposure to atmospheric water.

## SUMMARY OF THE INVENTION

We have found that activated solid acidic catalysts that have a metal oxide are good catalysts for alkylation of non-oxygen-containing aromatic hydrocarbons, such as benzene, toluene, xylene, or mixtures thereof, if the activated catalyst is used without exposure to atmospheric water. The metal oxide is selected from zirconium oxide or tin oxide, such as $ZrO_2$-$H_2SO_4$; $ZrO_2$-$WO_3$; and $SnO_2$-$H_2SO_4$. These catalysts are especially good for the alkylation of benzene, even when the olefins have sixteen or more carbon atoms per molecule. The prior art fails to recognize these metal oxide catalysts as good catalysts for this very difficult reaction.

The olefins have from 16 to 28 carbon atoms per molecule. Preferably, the olefins are alpha olefins having from 20 to 24 carbon atoms per molecule.

## DETAILED DESCRIPTION OF THE INVENTION

In its broadest aspect, the present invention involves the alkylation of non-oxygen-containing aromatic hydrocarbons, such as benzene and alkyl benzenes. In that alkylation reaction, aromatics are reacted with olefins in the presence of a solid, acidic catalyst comprising a metal oxide. That catalyst is activated prior to use and the activated catalyst is used without exposure to atmospheric water.

The olefins have from 16 to 28 carbon atoms per molecule are reacted in the presence of a solid, acidic catalyst that has either zirconium oxide or tin oxide. The preferred catalyst compositions are mixed oxides.

The alkylation of benzene or alkyl benzenes does not take place as readily as alkylation of other aromatics, and alkylation of aromatics with higher olefins in the $C_{16}$ to $C_{28}$ range does not take place as readily as with the lower olefins. The alkylation of benzene with higher olefins is especially difficult.

Because of environmental concerns, there is increased resistance to using hydrogen fluoride for alkylation of aromatics. The present invention is a solid, acid replacement for hydrogen fluoride. Solid, acidic catalysts have been used with low molecular weight olefins. They have not been used with higher molecular weight olefins, such as the $C_{20}$-$C_{24}$ $\alpha$-olefins of the present invention.

## NON-OXYGEN-CONTAINING AROMATIC HYDROCARBONS

The non-oxygen-containing aromatic hydrocarbon that is alkylated in the subject process is preferably benzene, but a higher molecular weight hydrocarbon may also be charged to the process. Benzene is less reactive than substituted aromatics, thereby it requires higher temperatures to get high conversion of product. The feed aromatic hydrocarbon may, therefore, be toluene, xylene, ethylbenzene, naphthalene, etc., as long as it doesn't contain oxygen.

## OLEFINS

The feed olefinic hydrocarbons that are consumed in the process may have sixteen to twenty-eight carbon atoms per molecule. Most preferably, they are predominately alpha olefins having from twenty to twenty-four carbon atoms per molecule. In this most preferred embodiment, small amounts of $C_{18}$ and $C_{26}$ olefins can be present.

## CATALYST

The catalysts of the present invention are solid, acidic catalysts that have a metal oxide selected from zirconium oxide or tin oxide. The catalyst is activated prior to use and the activated catalyst is used without exposure to atmospheric water.

The preferred catalyst compositions are mixed oxides. The preferred catalysts are $ZrO_2$-$H_2SO_4$; $ZrO_2$-$WO_3$; and $SnO_2$-$H_2SO_4$. The most preferred catalysts contain zirconium oxide, such as $ZrO_2$-$H_2SO_4$.

The preparation of zirconium oxysulfate catalysts is disclosed by King in U.S. Patent No. 4,873,017, entitled "Heterogeneous Alkoxylation Using Anion-Bound Metal Oxides.

Catalysts are activated by:

1) placing the catalyst in a suitable reservoir, reactor, or container capable of isolation from atmospheric moisture, water, or water vapor,

2) heating the catalyst to at least 75° C in the presence of a flow of dry, inert, non-protic material which will carry away with it water or other protic materials which desorb from the catalyst,

3) maintaining the flow of inert material to prevent contact of the activated catalyst with moisture or water, and optionally

4) heating or cooling the activated catalyst to a desired reaction temperature before the introduction of reactants.

The inert material may be dried gases such as nitrogen, helium, argon or dried liquids, such as hexane and benzene. The inert material must not be capable of, or contain materials, producing water or protic materials when heated or brought in the presence of an acidic catalyst (e.g., alcohols, ethers, ammonia, amines, etc.).

Preferably, the activation temperature is at least 100° C, because that is the boiling point of water. More preferred is about 150° C, but activation temperature has to be determined by experiment. In our practice, we have used 100° or 150° C activation for batch reactions and 150° to 300° C for flow reactions.

The catalyst can be bound by conventional means, such as disclosed by King in U.S. Patent No. 4,873,017 described above.

## EXAMPLES

The invention will be further illustrated by following examples, which set forth particularly advantageous method embodiments. While the Examples are provided to illustrate the present invention, they are not intended to limit it.

Examples 1 and 2 are two different preparations of catalysts of the present invention used in two different alkylation examples.

## EXAMPLE 1

## PREPARATION OF $ZrO_2$/$SO_4$ CATALYST A

$ZrOCl_2$•$8H_2O$ (150 g) was dissolved in 600 ml distilled water. To this solution was added concentrated ammonium hydroxide solution until the pH reached 10.0. As the ammonium hydroxide solution was added, a thick white precipitate formed. Additional water was added to aid mixing. The solids were collected on a filter and washed twice with distilled water. The solid material was dried for four days at 95-103° C. To the resulting hydrated zirconium hydroxide (9.661 g) from the previous step was added 151.8 ml 1 N (0.5 M) sulfuric acid. The mixture was stirred for half an hour and the solids were recovered by filtering through a fritted glass filter. The collected solids were washed twice with 100 ml of distilled water. The resulting solids were dried at 100° C for 18 hours. The dried solids were calcined in flowing air according to the following temperature schedule:

Heat to 120° C at 10°/min
Hold at 120° C for three hours
Heat to 540° C at 2°/min
Hold at 540° C for five hours
Heat to 585° C at 1°/min
Hold at 585° C for five hours
Cool to ambient room temperature.

## EXAMPLE 2

### PREPARATION OF $ZrO_2/SO_4$ CATALYST B

$ZrOCl_2 \cdot 8H_2O$ (184.68 g) was dissolved in 407.5 distilled water. To this solution was added 250 ml concentrated ammonium hydroxide solution in several portions over about eight minutes, causing the formation of a gelatinous precipitate. During the ammonium hydroxide addition, an additional 50 ml of water were added to help maintain stirring of the reaction mixture. At the end of the ammonium hydroxide addition the pH was 9.99. The solids were collected on a filter and washed twice with distilled water and were then partially dried on the filter. The solids were then dried in a 100° C oven for eighteen hours. To 75 g of the dried solids was added 1.125 L of 1 N (0.5 M) sulfuric acid. The solids were stirred for half an hour and collected by filtration. The collected solids were washed with 3000 ml of distilled water. The resulting solids were dried at 100° C for 18 hours. The dried solids were calcined in flowing air according to the temperature schedule given for Catalyst A.

## EXAMPLE 3

### BATCH TESTING FOR AROMATIC ALKYLATION WITH MINIMAL EXPOSURE TO ATMOSPHERIC WATER

One gram samples of catalyst were weighed into vials and placed in a 100° or 150° C oven for drying (activation temperature for 6 1/2 to 7 hours activation time). The vials were then rapidly removed from the oven and sealed by crimping on a serum cap. After cooling, 4 ml of feed (usually 4:1 aromatic:olefin molar basis) were added by syringing through the serum cap. The volume of feed was enough to completely cover the catalyst. Next, the old serum cap was removed and a new, unpunctured cap crimped on. The vials containing catalyst and feed were heated at the desired alkylation temperature for the desired alkylation time (usually 24 hours). At the end of the alkylation time, the vials and contents were cooled and the liquid products separated by filtration.

Alkylation reactions were analyzed by supercritical fluid chromatography (SFC) using a Lee Scientific chromatograph, equipped with a 10-meter X 100 micrometer i.d. SB-50 column and frit restrictor at 100° C. Peaks were detected by a flame ionization detector operating at 325° C. A carbon dioxide density ramp from 0.20-0.75 g/ml was used. Percent olefin conversion was measured as:

$$\frac{area\ olefin \times 100}{area\ olefin + area\ products}$$

| Example | Catalyst | Drying °C/Hrs | Batch Test °C/Hrs | %Olefin Conversion |
|---|---|---|---|---|
| 3A | $ZrO_2/SO_4$ | 100°C/5 hrs | 100°C/24 hrs | 88.1 |
| 3B | $ZrO_2/SO_4$ | 100°C/6 hrs | 100°C/18 hrs | 91.2 |
| 3C | $ZrO_2/WO_3$ | 100°C/6.5 hrs | 100°C/18 hrs | 78.1 |
| 3D* | $HfO_2/SO_4$ | 100°C/6 hrs | 100°C/24 hrs | 11.8 |
| 3E* | $TiO_2/SO_4$ | 100°C/6 hrs | 100°C/24 hrs | 25.1 |
| 3F* | $Fe_2O_3/SO_4$ | 100°C/6 hrs | 100°C/24 hrs | 11.6 |
| 3G | $SnO_2/SO_4$ | 100°C/6 hrs | 100°C/24 hrs | 41.0 |
| 3H* | $TiO_2/H_3PO_4$ | 100°C/6 hrs | 100°C/24 hrs | 9.9 |
| 3I* | $Al_2O_3/SO_4$ | 100°C/6.5 hrs | 100°C/24 hrs | 10.9 |

* Comparative

## EXAMPLE 4

## BATCH TESTING FOR AROMATIC ALKYLATION WITH EXPOSURE TO ATMOSPHERIC WATER.

Batch alkylation 4A and 4B experiments using a $ZrO_2$-$SO_4$ catalyst prepared identically to Catalyst B were conducted at 100° C as described in Example 3 except that activated catalysts were allowed to cool in un-sealed vials before addition of feed. Catalysts were activated at 100° C. 4ml feed and 1 g of catalyst were used. Control experiments 4C and 4D were done as in Example 3. Experiment 4D had a 6 ml feed.

| Experiment | % Olefin Conversion | Notes |
|---|---|---|
| 4A | 28.3 | Catalyst exposed to atmospheric water |
| 4B | 56.4 | Catalyst exposed to atmospheric water |
| 4C | 98.3 | Catalyst protected from atmospheric water |
| 4D | 97.0 | Catalyst protected from atmospheric water |

## EXAMPLE 5

## BENZENE ALKYLATION WITH CATALYST A

A 12.7 mm (1/2") OD reactor was packed with 17.3g of Catalyst A (pelletized and crushed to 20/40 mesh) with alundum placed above and below the catalyst bed. The catalyst was activated at 300°C under flowing nitrogen (100 standard $cm^3$/min) at atmospheric pressure for four hours. After cooling to ambient temperature under nitrogen, the catalyst was heated to 50°C and a feed consisting of benzene/$C_{20-24}$ $\alpha$-olefin (4:1 molar ratio) was introduced. After a pressure of 2.16 MPa (abs) (300 psig) was obtained, the flow rate was set at 15 $\mu$l/min. Product samples were taken periodically and analyzed as described above. After 28.9 hours, the temperature was increased to 75°C and after 53.8 hours, temperature was again increased to 100°C. At this temperature, a stable olefin conversion of 92% was obtained. At 149.6 hours, the flow rate was reduced to 11.83 $\mu$l/min. Temperature was raised to 115°C at 173.4 hours. From the period from 85.6 to 310.5 hours, conversion remained at least 92%. At 311.8 hours, the feed rate was increased to 35.5 $\mu$l/min. Conversion dropped significantly, so, at 342.0 hours, the feed rate was decreased to 23.66 $\mu$l/min. Conversion roughly held constant at about 80%. At 407.3 hours, the feed rate was decreased further to 7.10 $\mu$l/min. Eventually conversion increased again to about 90% conversion. This example demonstrates that alkylation of benzene with a $C_{20-24}$ $\alpha$-olefin can be accomplished at $\geq$ 90% olefin conversion at 100° to 115°C for over 200 hours.

## EXAMPLE 6

## BENZENE ALKYLATION WITH CALCINED CATALYST A

The catalyst from the previous experiment was removed from the reactor and calcined under flowing nitrogen according to the temperature schedule shown for the preparation of Catalyst A to remove organic residues and coke. It was then recharged to the reactor. It was activated at 300°C for three hours as described above. After cooling to ambient temperature, feed was introduced and pressure maintained at 2.16MPa (abs) (300 psig). The feed rate was set at 5.7 $\mu$l/min and the reactor was heated stepwise to 100°C. Conversion was increased to greater than 90% after two days at this temperature. After 239 hours at 100°, the temperature was increased to 115°. For a period of 290 hours at 100° or higher temperature, the olefin conversion was at least 88%.

## EXAMPLE 7

## NEED FOR ACTIVATION OF SULFATE ZIRCONIA CATALYST

Sulfated zirconia catalyst (1.784 g) was charged to a round-bottom flask equipped with a magnetic stirring bar and a reflux condenser. The condenser was air-cooled. To the round bottom flask were added 3.202 g of commercial $C_{20-24}$ alpha olefin and 3.907 g phenol. The molar ratio phenol:olefin was 4 and the weight ratio of feed (phenol + olefin) to catalyst was 4. The phenol and alpha olefin were used as received. The round bottom flask was heated by an oil bath pre-heated to 155° C while the contents of the flask were stirred magnetically. Samples were taken periodically by syringe and were analyzed by SFC. Results showed 99.4% olefin conversion in one hour. This experiment showed that phenol is readily alkylated by $C_{20-24}$ alpha olefin without any activation or drying of the catalyst.

The alkylation of benzene with $C_{20-24}$ alpha olefin was tested with and without prior catalyst activation by the method described in Example 3. In one experiment, sulfated zirconia catalyst (1.0 g) was added to a 30 ml bottle. Four ml of feed (4:1 benzene:alpha olefin) were added and the bottle sealed. The bottle was heated for 24 hours at 100° C. Analysis by SFC showed 14.4% olefin conversion. In a control experiment, 1 g of sulfated zirconia was added to a 30 ml bottle. The bottle and contents were heated at 100° C for 6 hours to activate the catalyst and then sealed while hot. After cooling, 4 ml of feed were added by syringe. The seal was replaced. The bottle and contents were heated at 100° C for 24 hours. Analysis by SFC showed 96.4% olefin conversion. These experiments show that activation of sulfonated zirconia catalyst for alkylation of non-oxygen containing aromatics by $C_{20-24}$ alpha olefin is necessary.

## Claims

1. A process for alkylation of non-oxygen-containing aromatic hydrocarbons, said process comprising reacting said aromatic hydrocarbons with olefins in the presence of a solid, acidic catalyst comprising a metal oxide selected from the group consisting of zirconium oxide and tin oxide, wherein said olefins have from 16 to 28 carbon atoms per molecule, wherein the catalyst is activated prior to use and wherein the activated catalyst is used without exposure to atmospheric water.

2. A process according to Claim 1 wherein the aromatic hydrocarbons are selected from the group consisting of benzene, toluene, xylene, and mixtures thereof.

3. A process according to Claim 2 wherein the aromatic hydrocarbons comprises benzene.

4. A process according to Claim 1 wherein the olefins are alpha olefins having from 20 to 24 carbon atoms per molecule.

5. A process according to Claim 1 wherein the catalyst comprises a zirconium oxide.

6. A process according to claim 1, wherein the catalyst is selected from the group consisting of $ZrO_2$-$H_2SO_4$; $ZrO_2$-$WO_3$; and $SnO_2$-$H_2SO_4$.

## Patentansprüche

1. Verfahren zur Alkylierung von nicht-sauerstoffhaltigen aromatischen Kohlenwasserstoffen, wobei das Verfahren umfasst das Umsetzen von aromatischen Kohlenwasserstoffen mit Olefinen in Gegenwart eines sauren Festkörper-Katalysators, beinhaltend ein Metalloxid, ausgewählt aus der Gruppe Zirkonoxid und Zinnoxid, wobei die Olefine 16 bis 28 Kohlenstoffatome pro Molekül enthalten, der Katalysator vor einer Verwendung aktiviert wird und der aktivierte Katalysator verwendet wird ohne Wasser der Atmosphäre ausgesetzt zu sein.

2. Verfahren nach Anspruch 1, wobei die aromatischen Kohlenwasserstoffe ausgewählt sind aus der Gruppe Benzol, Toluol, Xylol und deren Mischungen.

3. Verfahren nach Anspruch 2, wobei die aromatischen Kohlenwasserstoffe Benzol umfassen.

4. Verfahren nach Anspruch 1, wobei die Olefine Alpha-Olefine sind mit 20 bis 24 Kohlenstoffatomen pro Molekül.

5. Verfahren nach Anspruch 1, wobei der Katalysator ein Zirkonoxid enthält.

6. Verfahren nach Anspruch 1, wobei der Katalysator ausgewählt ist aus der Gruppe $ZrO_2 \cdot H_2SO_4$; $ZrO_2 \cdot WO_3$ und $SnO_2 \cdot H_2SO_4$.

## Revendications

1. Procédé pour l'alkylation d'hydrocarbures aromatiques ne contenant pas d'oxygène, ledit procédé comprenant la réaction desdits hydrocarbures aromatiques avec des oléfines en présence d'un catalyseur acide solide comprenant un oxyde métallique, choisi dans le groupe consistant en l'oxyde de zirconium et l'oxyde d'étain, lesdites oléfines ayant 16 à 28 atomes de carbone par molécule, dans lequel le catalyseur est activé avant utilisation et dans lequel le catalyseur activé est utilisé sans exposition à l'eau atmosphérique.

2. Procédé suivant la revendication 1, dans lequel les hydrocarbures aromatiques sont choisis dans le groupe consistant en le benzène, le toluène, le xylène et leurs mélanges.

3. Procédé suivant la revendication 2, dans lequel les hydrocarbures aromatiques comprennent le benzène.

4. Procédé suivant la revendication 1, dans lequel les oléfines sont des alpha-oléfines ayant 20 à 24 atomes de carbone par molécule.

5. Procédé suivant la revendication 1, dans lequel le catalyseur comprend un oxyde de zirconium.

6. Procédé suivant la revendication 1, dans lequel le catalyseur est choisi dans le groupe consistant en $ZrO_2$-$H_2SO_4$ ; $ZrO_2$-$WO_3$ et $SnO_2$-$H_2SO_4$.